(19) Europäisches Patentamt
European Patent Office
Office européen des brevets

(11) Publication number: 0 066 400 A1

(12) EUROPEAN PATENT APPLICATION

(21) Application number: 82302484.9

(22) Date of filing: 17.05.82

(51) Int. Cl.[3]: C 07 D 493/10
C 12 P 17/18, A 61 K 31/35
//(C07D493/10, 311/00, 303/00),
(C12P17/18, C12R1/645)

(30) Priority: 19.05.81 US 265135

(43) Date of publication of application: 08.12.82 Bulletin 82/49

(84) Designated Contracting States: AT BE CH DE FR GB IT LI LU NL SE

(71) Applicant: WARNER-LAMBERT COMPANY
201 Tabor Road
Morris Plains New Jersey 07950(US)

(72) Inventor: Bloem, Russell Jack
4117 Woodworth
Dearborn Michigan 48126(US)

(72) Inventor: Bunge, Richard Harold
35180 Dumbarton
Mt. Clemens Michigan 48045(US)

(72) Inventor: French, James Clark
3150 Rumsey
Ann Arbor Michigan 48105(US)

(74) Representative: Jones, Michael Raymond et al,
Haseltine Lake & Co. 28 Southampton Buildings
Chancery Lane
London WC2A 1AT(GB)

(54) **Antibiotic roridin L-2 and its production.**

(57) There is diclosed a novel trichothecene antibiotic, roridin L-2, and a process for its production. The compound roridin L-2 has the following formula:

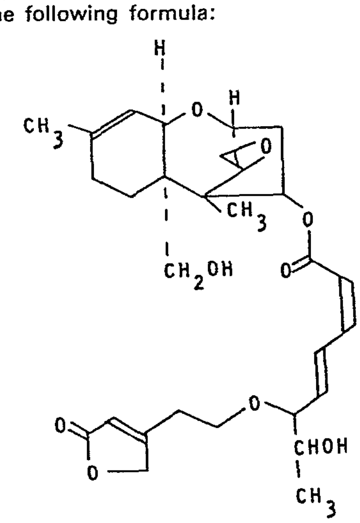

The compound roridin L-2, which may be formed by cultivating a suitable strain of *Myrothecium roridum*, NRRL 12303 has antimicrobial and antitumor activity.

EP 0 066 400 A1

Croydon Printing Company Ltd

"ANTIBIOTIC RORIDIN L-2 AND ITS PRODUCTION"

This invention relates to a novel trichothecene antibiotic compound, roridin L-2, which is represented by the following structural formula I, and to a process for the production of the compound:-

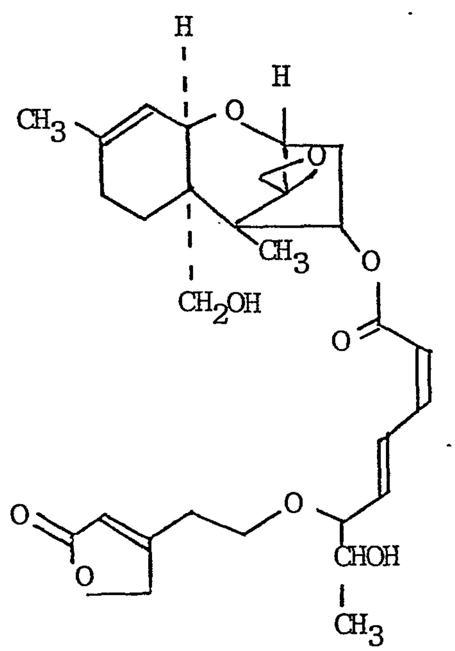

(I)

The process aspect of the present invention relates to a fermention process for the production of the compound of the present invention by cultivating a roridin L-2 producing strain of an organism belonging to the species Myrothecium roridum, especially a strain closely resembling Myrothecium roridum Tode.

In addition, the present invention provides pharmaceutical compositions containing in addition to a pharmaceutically acceptable carrier, the compound

of the invention alone or in combination with another trichothecene derivative or other antitumor agents in the treatment of bacterial infections and neoplastic diseases. A review of the trichothecene antibiotics is found in C. Tamm, "Progress in the Chemistry of Natural Products," 31, 64-117 (1974).

Mild alkaline hydrolysis of roridin L-2 produces verrucarol which has the following formula:-

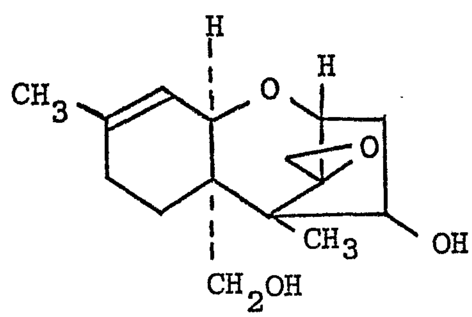

(II)

a dihydroxysesquiterpene which is a structural unit of several trichothecenes. Roridin L-2 can be readily distinguished from related, previously disclosed trichothecenes such as roridins A, D, E, E-2, H, J and K by the following characteristic properties exhibited by roridin L-2:

(1) an infrared absorption at 1780 $cm^{-1}$

(2) a proton magnetic resonance signal that appears, in deuterated chloroform solution, as a doublet (J = 2 Hz) centred at 4.80 ppm downfield from tetramethylsilane; and

(3) three $^{13}C$ magnetic resonance signals that appear, in deuterated chloroform solution, at 174.0, 167.4 and 166.5 ppm downfield from tetramethylsilane.

The above spectral properties are not shown by any of the known trichothecenes and clearly establish roridin L-2 as a new member of the trichothecene family of antitumor antibiotics.

In accordance with one aspect of the present invention, roridin L-2 is produced by cultivating a selected roridin L-2 producing strain of an organism that closely resembles Myrothecium roridum Tode under artificial conditions in a suitable nutrient medium until a substantial quantity of roridin L-2 is formed, which may then be isolated in pure form by procedures described hereinafter.

A new strain of a Myrothecium roridum species, suitable for the purpose of the present invention, has been isolated from a sample of soil collected in Cleveland, Ohio, United States of America. It corresponds morphologically to the description of this species by N.C. Preston, "Transactions Brit. Mycological Soc.," 26, 158 (1943); and J.C. Gilman, "Manual of Soil Fungi," Second Edition 1957, The Iowa State College Press, Ames, Iowa, United States of America. Cultures of this novel microbial strain have been deposited with the United States Department of Agriculture, Northern Utilization Research and Development Division, Peoria, Illinois, United States of America, and are being maintained in their permanent culture collection as NRRL 12303.

Cultural characteristics showed that the new Myrothecium roridum strain, NRRL 12303, differed slightly from the reference Myrothecium roridum Tode in the aerial and submerged mycelia.

The following table describes the mycelial characteristics of the two organisms in the different standard microbiological media: PDA (Potato Dextrose Agar), SDA (Sabouraud's Dextrose Agar), CZP (Czapek Dox Agar), and BHI (Brain Heart Infusion Agar).

Cultural Characteristics of 7-Day Cultures

| Type of Mycelia | Media | Strain NRRL 12303 | Myrothecium roridum Tode |
|---|---|---|---|
| | PDA | Very variable:white, fluffy, slight yellow to dark green or black | Black with white edges |
| Aerial Mycelia | SDA | White to orange yellow to dark green or black | White to slight pink-yellow, to dark green or black |
| | CZP | White to pink with black tufts | White to slight pink with black tufts |
| | BHI | White to slight pink | White to slight pink |
| | PDA | Yellow orange to greyish green | Dark green with orange yellow edges |
| Submerged Mycelia | SDA | Yellow orange with tufts of black | Yellow orange with tufts of black |
| | CZP | Yellow orange with tufts of black | Black with yellow orange edge |
| | BHI | Orange yellow | Yellowish orange |

The antitumor antibiotic compound, roridin L-2, is produced by the fungus during aerobic fermentation under controlled conditions. The fermentation medium consists of suitable sources of carbon, nitrogen, inorganic salts, and growth factors assimilable by the microorganism. Examples of carbon sources are various sugars such as cerelose, lactose, and maltose; starch; dextrin; corn meal; and glycerol. The quantity of the carbon sources usually is in the range from 0.5 to 6% by weight, but levels outside this range can also be used.

The sources of nitrogen can be organic, inorganic, or mixed organic-inorganic in nature. Examples of nitrogen sources that can be used in the culture medium are cottonseed meal, corn germ flour, soybean meal, corn steep liquor, distillers' solubles, peanut meal, fish meal, peptonized milk, and various ammonium salts. The amount added usually is in the range from 0.1 to 3%, but higher amounts are also acceptable.

The inclusion of certain amounts of minerals and growth factors in the fermentation medium is also helpful in the production of roridin L-2. Crude medium ingredients such as distillers' solubles, corn steep liquor, fish meal, yeast products, peptonized milk, and whey contain minerals and growth factors. However, inorganic salts such as potassium phosphate, sodium chloride, ferric sulphate, calcium carbonate, colbalt chloride, and zinc sulphate can be added to the fermentation medium.

The preferred process for producing roridin L-2 by using Myrothecium roridum is by submerged fermentation. According to this preferred process of the present invention, fermentation ingredients are prepared in solution and sterilized by autoclaving or steam heating. The pH of the aqueous medium is preferably in the range from 6 to 8. The fermentation medium is cooled to a suitable temperature, generally in the range from

20 to 45$^{\circ}$C, and then inoculated with the suitable culture. Fermentation is carried out with aeration and agitation, and the maximum production of roridin L-2 is usually reached in three to eight days.

In the submerged culture method, fermentation is carried out in shake flask or in stationary vat fermentors. In shake flasks, aeration is brought about by agitation of the flask which causes mixing of the medium with air. In the stationary fermentors, agitation is provided by impellors in the form of a disc turbine, vaned disc, open turbine, or marine propeller; and aeration is accomplished by injecting air or oxygen into the fermentation mixture.

The examples which follow illustrate the preferred methods by which the product, roridin L-2, of the present invention may be obtained. The described process is capable of wide variation, and the present invention is not limited to the described process.

Fermentation in 200-Gallon ( 757 litre) Fermentors

A. Seed Development

A culture of the microorganism *Myrothecium roridum* NRRL 12303, preserved in a soil tube, was transferred to CIM 23 agar slants and incubated at 28$^{\circ}$C for seven days.

CIM-23 Slant Medium

| | |
|---|---|
| Amidex corn starch | 10 g |
| N-Z Amine, type A | 2 g |
| Beef extract (Difco) | 1 g |
| Yeast extract (Difco) | 1 g |
| Cobalt chloride $\cdot 6H_2O$ | 20 mg |
| Agar | 20 g |
| Distilled water | 1,000 ml |

The microbial growths in two slants were scraped, suspended in distilled water, and used to inoculate a 30 litre seed fermentor. The seed fermentor was prepared by charging it with 16 litres of ARM 1558A

medium and then autoclaving for 90 minutes at 121°C and 15 pounds per square inch gauge (103.4 kiloPascals).

ARM 1558A

| | |
|---|---|
| Cerelose | 2.0% |
| Pharmamedia | 0.3% |
| Defatted corn germ flour | 0.1% |
| Soybean meal | 0.1% |
| $CaCO_3$ | 1.0% |
| $K_2SO_4.7H_2O$ | 0.1% |
| $MgSO_4.7H_2O$ | 0.1% |
| NaCl | 0.05% |
| $FeSO_4.7H_2O$ | 0.0001% |

Use tap water, no pH adjustment.

B. Production Fermentation

Two 220 gallon ( 757 litre) fermentation tanks were used for producing roridin L-2. Each of these fermentors contained 160 gallons (606 litres) of presterilized ARM 1558 medium.

ARM 1558 Medium

| | |
|---|---|
| Cerelose | 5.0% |
| Pharmamedia | 0.3% |
| Defatted corn germ flour | 0.1% |
| Soybean meal | 0.1% |
| $CaCO_3$ | 1.0% |
| $K_2HPO_4$ | 0.1% |
| $MgSO_4.7H_2O$ | 0.1% |
| NaCl | 0.05% |
| $FeSO_4.7H_2O$ | 0.0001% |

Use tap water, no pH adjustment.

Each fermentor was inoculated with about 15 litres of the microbial growth from a 30 litre seed fermentor. The inoculated fermentors were stirred at 190 rpm at 30°C and sparged with 20 CFM (cubic feet per minute, equivalent to 566 litres per minute) of air for 119 hours. Dow Corning antifoam "C" was used to control foaming as required.

Production of roridin L-2 in the fermentation

broth was assayed vs KB and L1210 tissue cell lines. An aliquot of the fermentation broth was mixed with growing cultures of KB and L1210 cells to give a final dilution of the fermentation broth of 1:100. Activity was expressed as the percent growth of the cells relative to the control (no fermentation broth added). A fermentation broth that gave percent growth values of 0-25% for KB and 0-50% for L1210 was considered active.

The activities of the fermentation broths after 119 hours of fermentation are:

| Inhibitory Zone (mm) | | Cytotoxicity (% growth) | |
|---|---|---|---|
| Saccharomyces cerevisiae | Candida albicans | L1210 | KB |
| 16 | 15 | 4 | 0 |
| 15 | 17 | 4 | 9 |

Isolation of Roridin L-2

The fermentation beers (a total of 1350 litres) from two 200 gallon ( 757 litre) fermentors prepared as described above were combined and mixed vigourously with 675 litres of ethyl acetate at pH 6.6 for one hour. Celite 545 (91 kg) was then added and the stirred mixture was filtered using a 75.8 cm plate and frame filter press. The filter cake was washed with 245 litres of ethyl acetate followed by 188 litres of deionized water. The filtrate and filter cake washes are combined and allowed to stand to permit the separation of phases. The upper organic phase amounted to about 660 litres; the lower aqueous phase (1520 litres) was separated and mixed with 340 litres of fresh ethyl acetate. After standing, the layers were separated and the upper organic layer (356 litres) was added to the first ethyl acetate extract. This combined ethyl acetate extract (1015 litres) was then concentrated *in vacuo* to 13.2 litres. The concentrated extract was dried using anhydrous sodium sulphate and then filtered. The solvent was removed *in vacuo* to leave approximately 900 g of an oily residue. This product,

hereinafter referred to as residue A, was processed by the fermentation method described below to afford a pure sample of roridin L-2.

Purification Method

A solution of 25g of residue A in 25 ml of methylene chloride was fractionated using a Prep LC/System 500 apparatus fitted with one Prep Pak 500 /silica gel cartridge (5.7 cm x 30 cm), both of which are available from Waters Instruments, Inc., Milford, Massachusetts, U.S.A. After injection of the charge, the silica gel column was eluted consecutively with 2000 ml of methylene chloride, 2500 ml of 75:25 methylene chloride:ethyl acetate, 3000 ml of 50:50 methylene chloride:ethyl acetate, 1000 ml of 25:75 methylene chloride:ethyl acetate and finally with 1000 ml of ethyl acetate. Analysis of each fraction by means of high performance liquid chromatography (HPLC) using a 0.39 cm (I.D.) x 30 cm μ Bondpak ($C_{18}$ silica gel) column, a solvent system consisting of 60:40 methanol:water, and UV detection at 254 nm showed that most of the fractions eluted with 50:50 methylene chloride:ethyl acetate contained the desired material, roridin L-2. The retention time of this compound in the above analytical HPLC system was approximately 3.2 minutes using a flow rate of 2 ml/ minute. The fractions (2000 ml) containing roridin L-2 were combined and concentrated *in vacuo* to leave an oily residue, residue B (4.2 g).

Residue B was combined with 1.8 g of equivalent material obtained from a previous Prep LC fractionation and dissolved in 21 ml of 70:30 methanol:water. One-third portions of this solution were then subjected tp further fractionation by low pressure chromatography over 250 g of $C_{18}$ silica gel (Waters Instrument, Inc.), packed in a 4.8 cm (O.D.) x 45 cm Michel-Miller column (available from Ace Glass Co., Vineland, New Jersey, U.S.A.) using an eluant consisting of 50:50

methanol:water. Between chromatographic runs, the $C_{18}$ silica gel column was washed with methanol and reequilibrated with 50:50 methanol:water before the next charge was introduced. Fractions were monitored using the analytical HPLC conditions described above. The fractions that contained only roridin L-2 were combined and concentrated *in vacuo* to remove methanol. The remaining aqueous phase was extracted with two 500 ml portions of ethyl acetate. The organic extracts were combined, washed with 200 ml of water, dried over anhydrous sodium sulphate, and then filtered. Removal of the ethyl acetate by concentration of the filtrate *in vacuo* afforded 3.4 g of pure roridin L-2 as a white solid. The properties which defined roridin L-2 as a novel compound are listed below.

Properties of Roridin L-2

Ultraviolet Absorption Spectrum in Methanol, as represented in Figure 1 of the accompanying drawings:-

| $\lambda_{max}$ | $E_{1cm}^{1\%}$ |
|---|---|
| 259 nm | 465 |

Infrared Absorption Spectrum in KBr, as represented in Figure 2 of the accompanying drawings:-

Principal absorptions at: 3450, 2970, 1782, 1750, 1640, 1600, 1435, 1284, 1180, 1084, 1030 and 965 reciprocal centimetres.

Optical Rotation:-

$[\alpha]_D = 83.6^o$ (1.0% in chloroform)

Elemental Analysis:-

1. dried 18 hours at 25°C/ 1.33 Pascals

| | % C | % H | % O |
|---|---|---|---|
| Calcd. for $C_{29}H_{38}O_9 \cdot 0.5\ H_2O$ | 64.55 | 7.29 | 28.17 |
| Found: | 64.68 | 7.12 | 28.70 |
| | 64.41 | 7.15 | 28.95 |

2. dried for 2 hours at 100°C/ 13.3 Pa and then for 4 hours at 50°C/ 13.3 Pa

| | % C | % H |
|---|---|---|
| Calcd. for $C_{29}H_{38}O_9$: | 65.64 | 7.22 |
| Found: | 65.21 | 6.93 |
| | 65.36 | 7.28 |

Proton Magnetic Resonance Spectrum, 2% in $CDCl_3$, as represented in Figure 3 of the accompanying drawings:

Principal signals at:

(s = singlet, d = doublet, t = triplet, m = multiple) 0.83s, 1.16d, (J = 6), 1.72s, 2.02m, 2.81d (J = 4), 3.13d (J = 4), 3.5-4.0 (complex signals), 4.80d (J = 2), 5.49 broad d (J = 6), 5.22-6.02 (complex signals), 6.08 dd (J = 4,6), 6.60t (J = 11), and 7.60 dd (J = 11,15) parts per million downfield from tetramethylsilane.

Carbon-13 Nuclear Magnetic Resonance Spectrum in $CDCl_3$, as represented in Figure 4 of the accompanying drawings:-

The principal signals, in parts per million downfield from TMS, are at:

| Signal Number | Ppm (downfield from TMS) | Signal Number | Ppm (downfield from TMS) |
|---|---|---|---|
| 1 | 173.95 | 18 | 73.46 |
| 2 | 167.43 | 19 | 69.74 |
| 3 | 166.46 | 20 | 66.83 |
| 4 | 143.37 | 21 | 66.34 |
| 5 | 140.57 | 22 | 65.59 |
| 6 | 138.89 | 23 | 62.51 |
| 7 | 130.53 | 24 | 48.86 |
| 8 | 118.88 | 25 | 48.00 |
| 9 | 118.72 | 26 | 44.28 |
| 10 | 116.67 | 27 | 36.19 |
| 12 | 85.38 | 28 | 29.23 |
| 13 | 78.91 | 29 | 28.04 |
| 14 | 78.42 ) | 30 | 23.19 |
| 15 | 77.02 ) $CDCl_3$ | 31 | 21.14 |
| 16 | 75.62 ) | 32 | 18.44 |
| 17 | 75.40 | 33 | 6.57 |

High Performance Liquid Chromatography

| | |
|---|---|
| Column: | μ Bondpak $C_{18}$ (3.9 mm I.D. X 30 cm) |
| System: | 60:40 methanol:water |
| Flowrate: | 2 ml/min |
| Detection: | Ultraviolet absorption at 254 nm |
| Retention time: | 3.2 min |

Antitumor Activity of Roridin L-2 Against P388 Lymphatic Leukemia in Mice

| Dose (mg/kg/day) | T/C* percent MST | |
|---|---|---|
| | test 1 | test 2 |
| 16 | --- | 185 |
| 8 | 157 | 176 |
| 4 | 134 | 132 |
| 2 | 140 | 144 |
| 1 | 132 | 150 |

*T/C percent MST = 100 x median survival time in days of treated/control mice. Values 130 were considered active. The test method used was based on that described in Cancer Chemother. Reports 3: 1-87 (part 3), 1972.

The $ID_{50}$ of Roridin L-2 against L1210 cells was 0.178 μg/ml. The zone diameter of inhibition of Roridin L-2 against KB cells in agar was 28 mm when a 6.4 mm paper disc moistened with a solution containing 100 μg of Roridin L-2 per millilitre was used; and 12 mm when a 6.4 mm paper disc moistened with a solution containing 10 μg of Roridin L-2 per millilitre was used.

The antibiotic roridin L-2 can be used for its antimicrobial and antitumor activity in the form of pharmaceutical compositions containing roridin L-2 and a compatible pharmaceutically acceptable carrier. The compositions may also contain other active antibacterial and/or antitumor agents. The

compositions may be made up in any pharmaceutical form appropriate for the route of administration intended. Examples of such compositions include solid compositions for oral administration such as tablets, capsules, pills, powders and granules, liquid compositions for topical or oral administration such as solutions, suspensions, syrups and elixirs, and preparations for parenteral administration such as sterile solutions, suspensions, or emulsions.

For use as an antibacterial agent, the compositions are administered so that the concentration of active ingredient is greater than the minimum inhibitory concentration for the particular organism being treated. A suggested dosage regimen for use as an antitumor agent in mammalian species is 0.1 to 10 $mg/m^2$ for a single daily intravenous treatment course with roridin L-2. (The surface area of an adult male is approximately 1.7 square metres).

CLAIMS (for all States except Austria):

1. The antibiotic compound roridin L-2 having the following structural formula:-

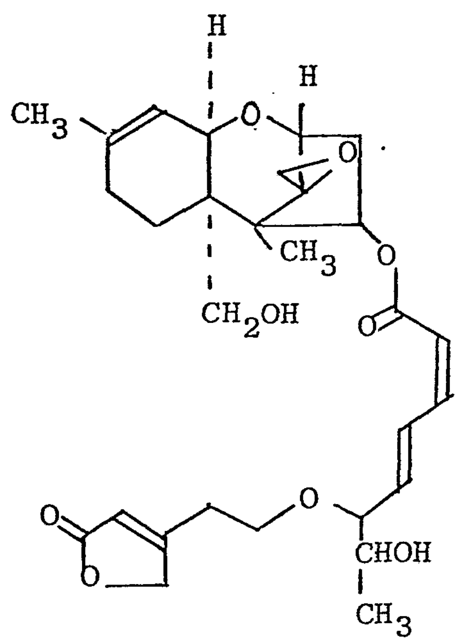

(I)

2. A process for the production of the compound roridin L-2 as claimed in Claim 1, which process comprises cultivating a roridin L-2 producing strain of the organism Myrothecium roridum in a suitable medium until a substantial quantity of roridin L-2 is formed.

3. A process according to Claim 2, which further includes the step of isolating the compound roridin L-2 in pure form.

4. A process according to Claim 2 or 3, wherein the strain of Myrothecium roridum is that deposited as NRRL 12303.

5. A pharmaceutical composition comprising the antibiotic compound roridin L-2 as claimed in Claim 1, and a pharmaceutically acceptable carrier.

CLAIMS (for Austria only):

1. A process for producing the antibiotic compound roridin L-2 which has the following structural formula:

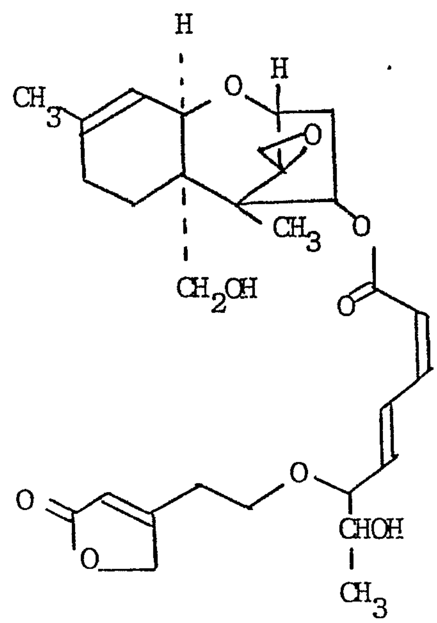

(I)

which process comprises cultivating a roridin L-2 producing strain of the organism Myrothecium roridum in a suitable medium until a substantial quantity of roridin L-2 is formed.

2. A process according to Claim 1, which further includes the step of isolating the compound roridin L-2 in pure form.

3. A process according to Claim 1 or 2, wherein the strain of Myrothecium roridum is NRRL 12303.

4. A process for producing a pharmaceutical composition, which comprises incorporating into a pharmaceutically acceptable carrier the compound roridin L-2 of formula I shown in Claim 1.

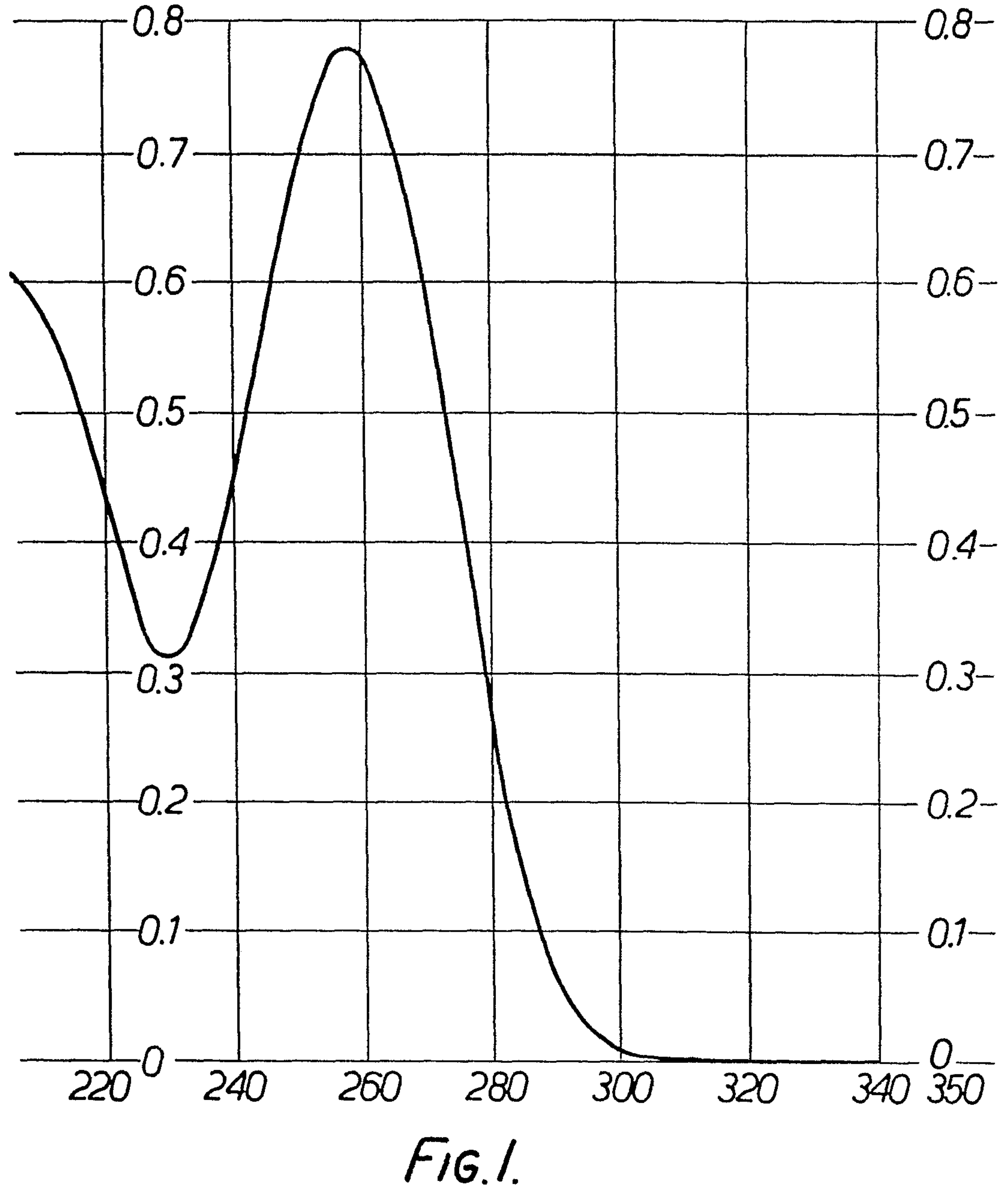


FIG. 1.

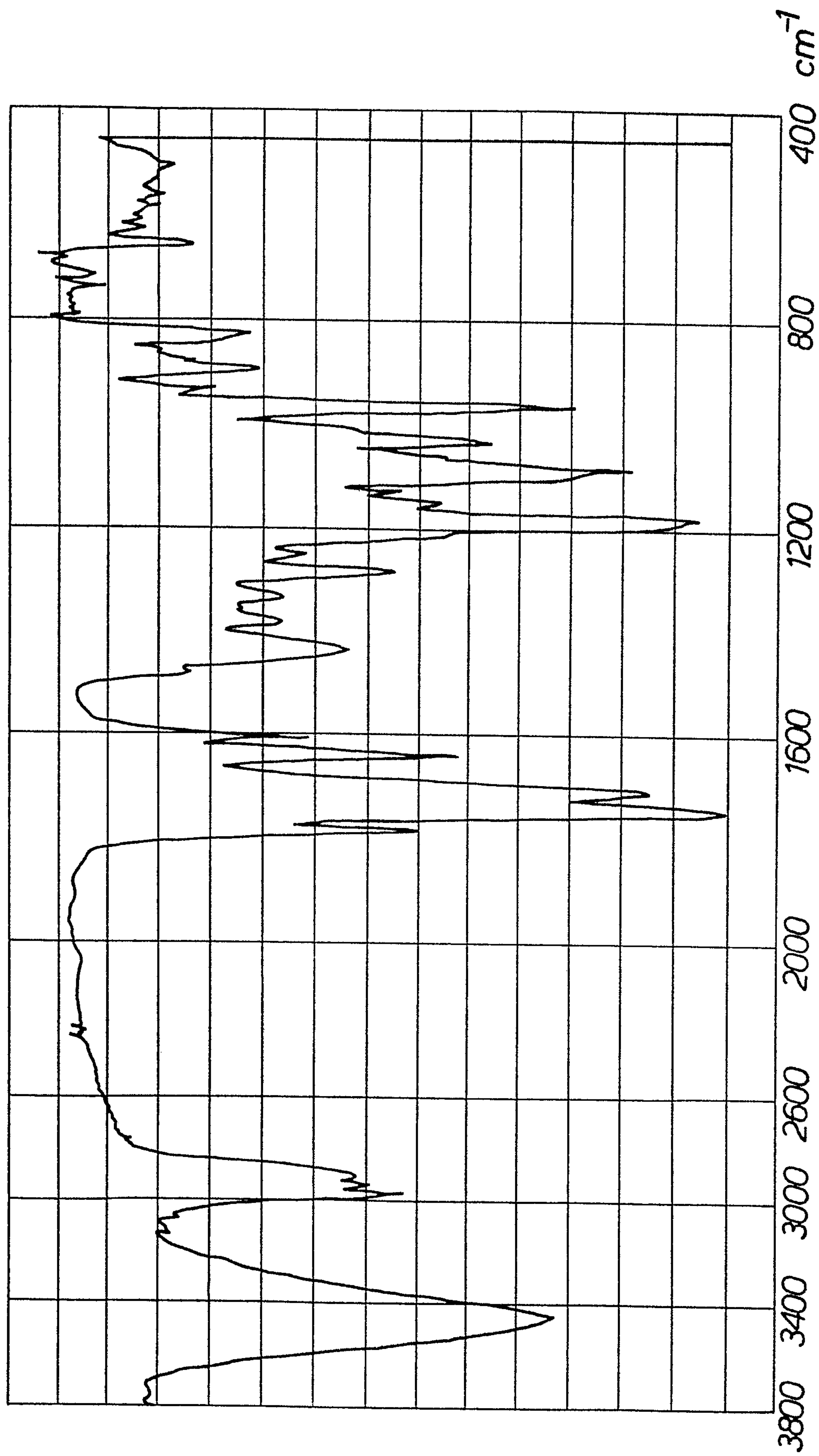


FIG. 2.

0066400

3/4

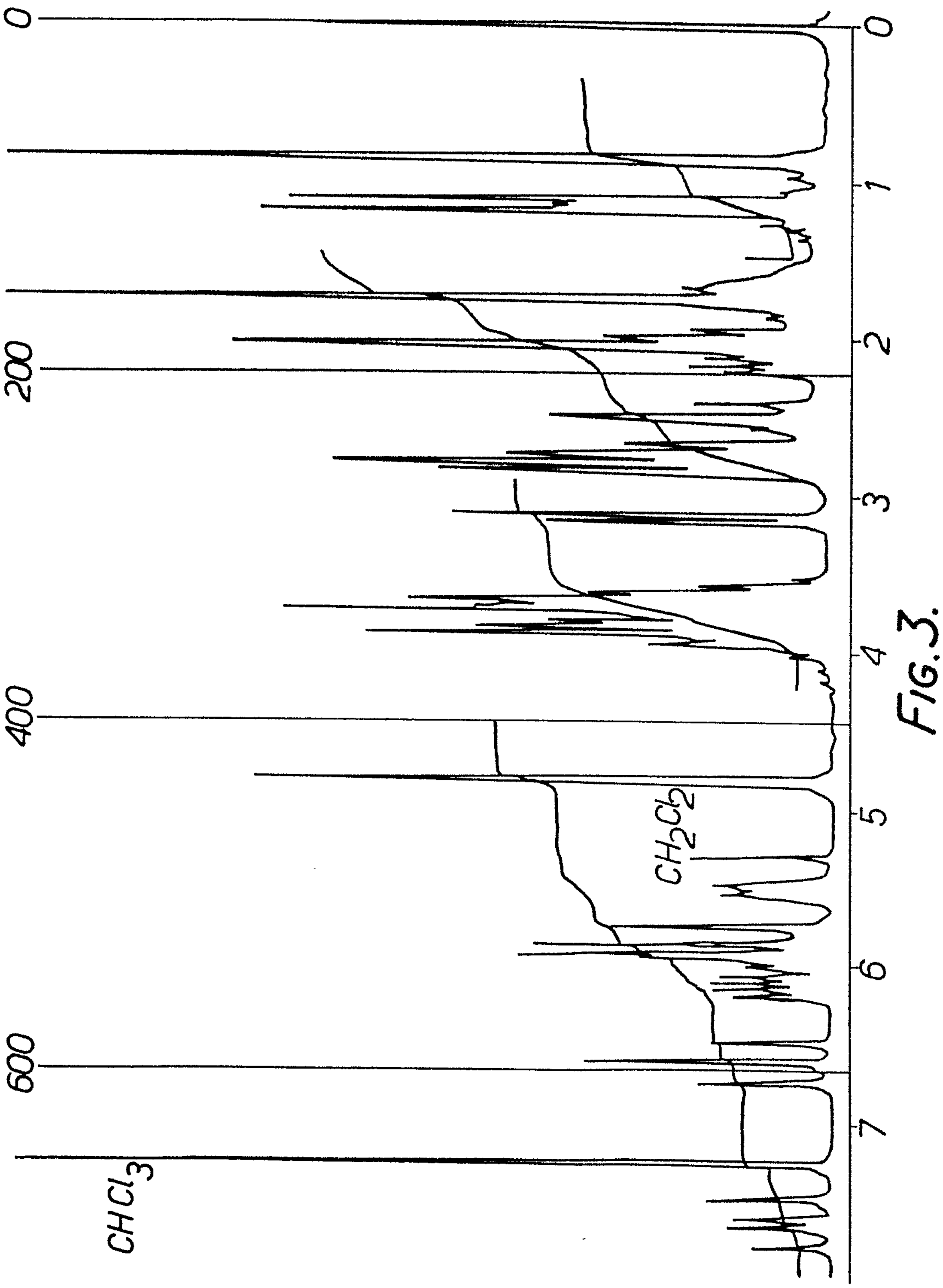


FIG. 3.

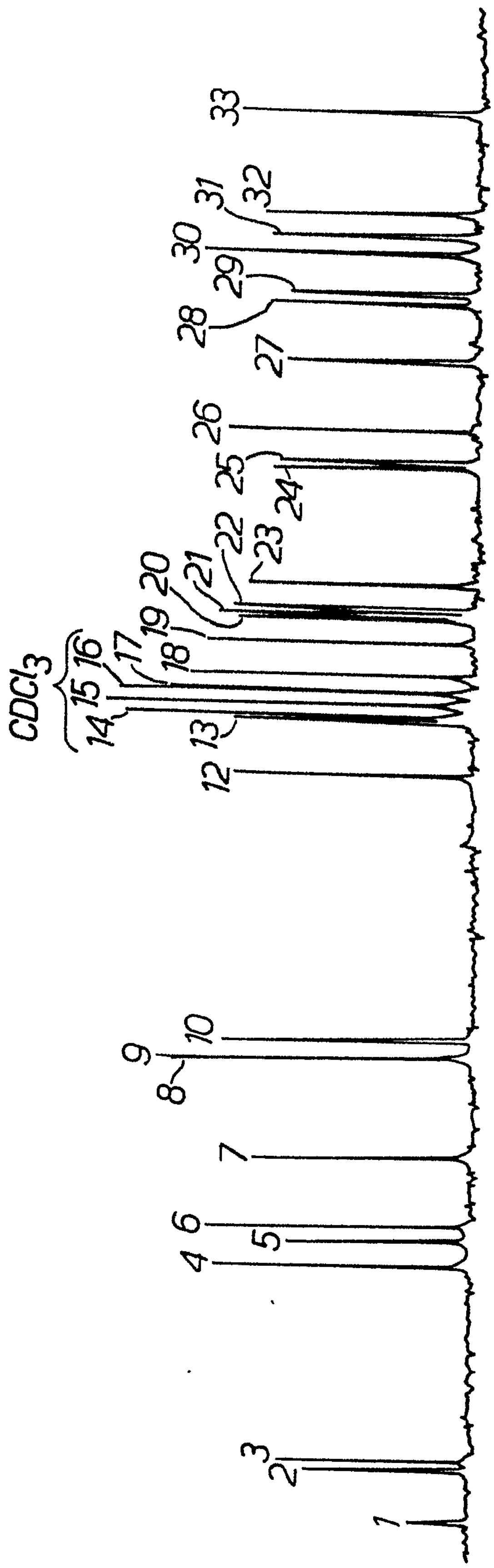

CDCl3
1
2
3
4
5
6
7
8
9
10
12
13
14
15
16
17
18
19
20
21
22
23
24
25
26
27
28
29
30
31
32
33


FIG. 4.

0066400

European Patent Office

# EUROPEAN SEARCH REPORT

Application number

EP 82 30 2484

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,X | THE JOURNAL OF ORGANIC CHEMISTRY, vol. 47, no. 6, 12th March 1982, pages 1117-1123; B.B.JARVIS et al.: "Isolation and characterization of the trichoverroids and new Roridins and verrucarins". *Page 1117,1118, "Chart I"; page 1119, "Chart II"; page 1121, line 12 from below and formula "i"* --- | 1-5 | C 07 D 493/10<br>C 12 P 17/18<br>A 61 K 31/35 //<br>(C 07 D 493/10<br>C 07 D 311/00<br>C 07 D 303/00 )<br>(C 12 P 17/18<br>C 12 R 1/645) |
| A | FR-A-1 348 003 (SANDOZ) *The whole document* --- | 1,2,5 | |
| A | US-A-4 244 874 (KANEKO) *The whole document* ----- | 1 | |
| | | | TECHNICAL FIELDS SEARCHED (Int. Cl. 3) |
| | | | A 61 K 31/00<br>C 07 D 493/00<br>C 12 P 17/00<br>C 12 R 1/00 |

The present search report has been drawn up for all claims

| Place of search | Date of completion of the search | Examiner |
|---|---|---|
| THE HAGUE | 12-08-1982 | RAJIC M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82